# EUROPEAN PATENT APPLICATION

(11) **EP 0 614 079 A2**
(43) Date of publication of application: **07.09.1994**
(21) Application number: 94250045.5
(22) Date of filing: 23.02.1994
(51) Int. Cl.: G01N 33/18, G01N 21/85, G01N 21/31, C12Q 1/18, C02F 1/50

(54) **A method of directly monitoring the concentrations of microbiocides in aqueous systems**

(30) Priority: 03.03.1993 US 25693
(71) Applicant: W.R. Grace & Co.-Conn., New York, New York 10036 (US)
(72) Inventor: Tully, Jack C., Wauconda, Ill .60084 (US); Kye, Larry M., Hoffman Estates, Ill.60195 (US)
(74) Representative: UEXKÜLL & STOLBERG

(57) **Abstract**

A method for directly measuring the concentration of biocides in aqueous systems comprising directly determining an absorbance or emission spectrum of the aqueous system in a wavelength range of from 200 to 2500 nm and applying chemometrics algorithms to the spectrum to determine the concentration of the biocides.

## Description

### FIELD OF THE INVENTION

The present invention is directed to a method for directly monitoring the concentration of biocides in aqueous systems and more particularly to a direct spectrometric method for quantifying biocides which have an absorbance or emission spectrum in a wavelength range of from 200 to 2500 nm wherein the spectrum of the aqueous system containing the biocides is determined and chemometric algorithms are applied to the spectrum.

### BACKGROUND OF THE INVENTION

Traditional water treatment analysis methods involve taking grab samples and performing independent analytical procedures for each component of interest. Typically these are time consuming and involve significant delay between taking samples, obtaining results and finally making program adjustments. Some recent on-line analysis techniques have been developed, but these techniques are either not specific to a particular analyte, they are limited to measuring single components or they require the use of addition of reagents to develop a color intensity which is proportional to the concentration of the analyte of interest. For example, on-line analyzers have been developed which are capable of monitoring oxidizing biocides, i.e., ORP - Oxidation Reduction Potential analyzers. However, these analyzers are not specific and will respond to the presence of any oxidizing compounds in the system. Colorimetric analysis are similarly deficient due to:
1. Slow response time since most colorimetric reactions take several minutes to develop.
2. Colorimetric reactions are subject to interference from background contaminants and physical parameters. For example many colorimetric endpoints are sensitive to temperature and pH.
3. Maintenance requirements. Periodic reagent replacement and re-standardization.

Another technique that has been used to monitor aqueous systems relies on the measurement of inert tracer components to indirectly monitor product levels. However, active biocides which are used to treat water treatment systems are not inert and are consumed or degraded under normal operating conditions within the aqueous systems. For this reason periodic sampling of the active biocidal agents must still be made to ensure system protection.

Thus, there exists a need for a rapid, direct method of monitoring active biocide concentrations in aqueous systems.

### SUMMARY OF THE INVENTION

It is an object of the present invention to provide a method for the simultaneous direct measurement of active concentrations of one or more biocides in an aqueous system and which does not require chemical reagents and is generally unaffected by the presence of background interferences.

It is another object of the present invention to provide a method for a simultaneous analysis and feed-back to a control system to maintain and adjust biocide feed rates in an aqueous system.

It is another object of the present invention to provide a method for the direct and simultaneous determination of active biocide levels and tracer levels in an aqueous system to determine overall treatment performance.

It is yet another object of the present invention to provide a method for the identification and quantification of low levels of weak UV-vis-NIR absorbing biocides in the presence of stronger absorbing UV-vis-NIR water treatment agents which could not heretofore be quantified by conventional UV-vis-NIR spectrometrics techniques.

In accordance with the present invention there has been provided a method for measuring the concentration of one or more biocides in an aqueous system with a unique combination of UV-vis-NIR spectrometry with the application chemometrics algorithms to determine active biocide levels in the aqueous system.

### BRIEF DESCRIPTION OF THE DRAWINGS

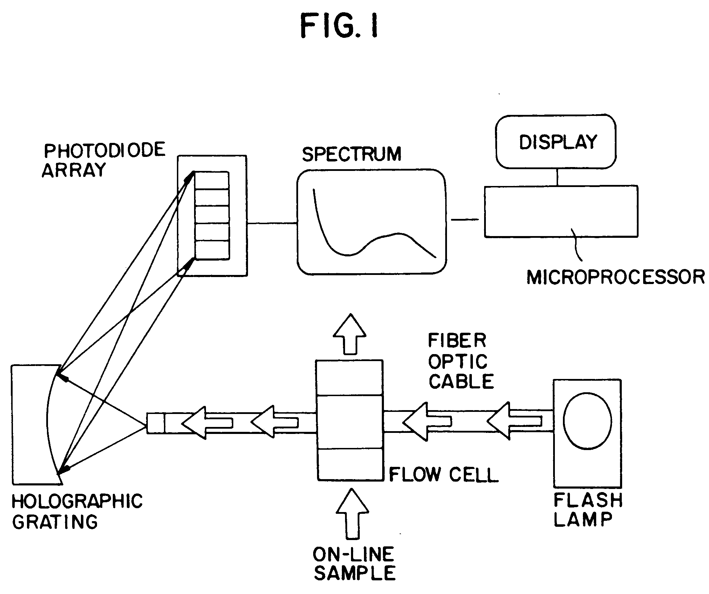

Figure 1 is a schematic of an on-line analyzer.

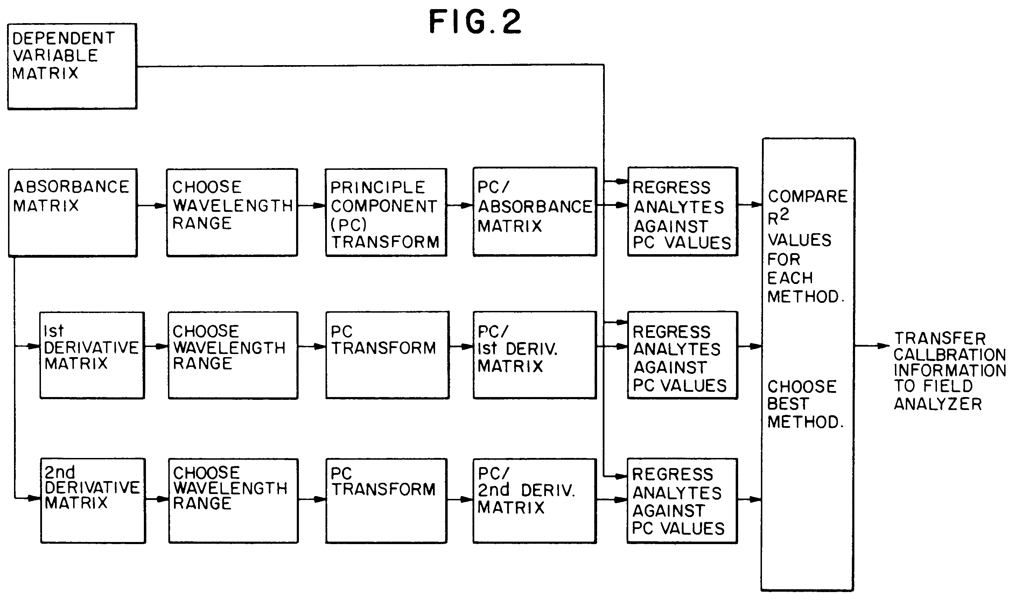

Figure 2 is a schematic of a multisample laboratory calibration.

Figure 3 is a graph showing actual 5-chloro-2-methyl-4-isothiazoline-3-one levels versus calibration predictions for a learning set used in a trial on a synthetic cooling tower. The diagonal line in the figure represents the perfect match and the absolute error of a prediction is represented by the vertical distance between the line and the predicted point.

Figure 4 is a graph of HPLC results versus the corresponding analyzer readings for the synthetic cooling tower trial grab samples of 5-chloro-2-methyl-4-isothiazoline-3-one.

Figure 5 is a line plot of analyzer readings and HPLC results (filled circles) for the concentrations of 5-chloro-2-methyl-4-isothiazoline-3-one in the synthetic cooling tower study.

Figure 6 is a graph showing actual 2,2-dibromo-3-nitrilpropionamide levels versus calibration predictions in a trial on a synthetic cooling tower. The diagonal line in the figure represents the perfect match and the absolute error of a prediction is represented by the vertical distance between the line and the predicted point.

Figure 7 is a graph of titration vs the corresponding analyzer readings for a synthetic cooling tower trial containing 2,2-dibromo-3-nitrilpropionamide.

Figure 8 is a line plot of analyzer readings and titration results (filled circles) for the concentrations of 2,2-dibromo-3-nitrilpropionamide in the synthetic cooling tower study.

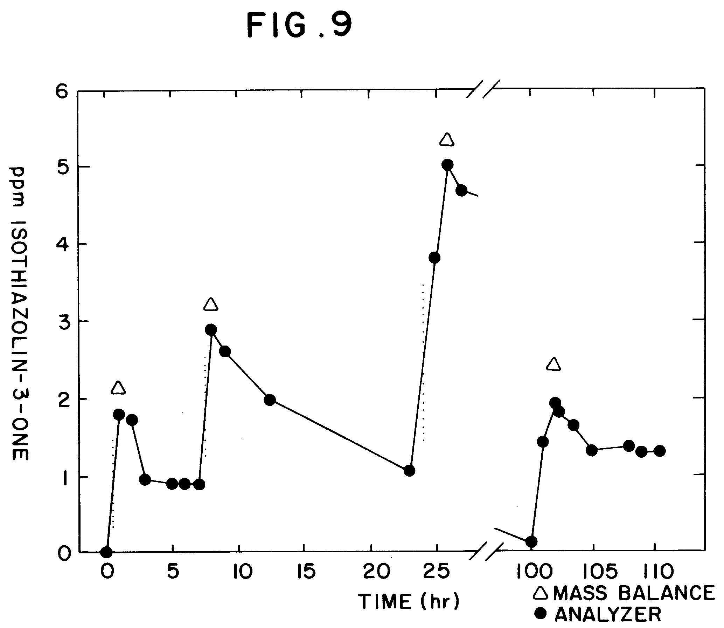

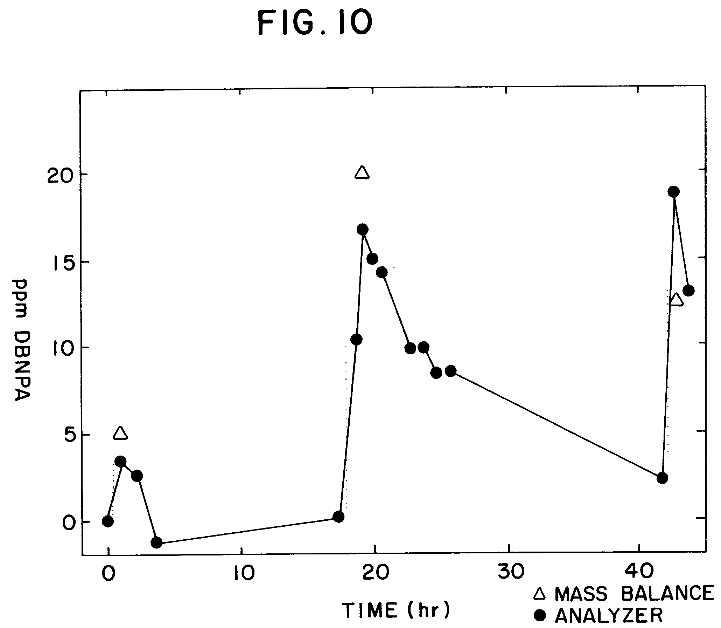

Figures 9 and 10 are graphical summaries of field results where concentrations of 5-chloro-2-methl-4-isothiazoline-3-one and 2,2-dibromo-3-nitrilpropionamide are plotted against mass balance concentrations.

### DETAILED DESCRIPTION

The present invention is directed to a novel method for directly monitoring the concentrations of one or more biocides in an aqueous system. The method, in general, involves directly determining an absorbance or emission spectrum of the system water containing the biocide(s) in the ultraviolet, visible and/or near infrared wavelength range, and then applying chemometrics algorithms to the spectrum to determine the concentration(s) of the biocide(s). The method of the present invention is generally unaffected by the presence of background matrix interferences in the system water such as pH changes or the presence of other active water treating components, and thus does not require time consuming, off-line separation or derivatization techniques. In addition, the method of the present invention does not require or involve the use of additional colorizing agents, dyes, titrations or other indirect monitoring techniques.

The present invention is of general applicability with respect to biocides which are presently used to treat aqueous systems, provided of course, that the particular biocide(s) of interest has a detectable absorbance or emission spectrum in the ultraviolet, visible and/or near infrared range (i.e. in the wavelength range of from 200 to 2500 nm). For purposes of this invention, a biocide is considered detectable if it has a chromophore with at least about 0.1 absorbance units (or a corresponding measurable response with an emissivity spectrometer) in the wavelength range of from 200 to 2500 nm under normal biocide treatment dosages. It is preferred that the biocide has between 0.1 to 1.5 absorbance units in the above wavelength range and dosage amounts.

Examples of biocides that have been found to be easily monitorable in accordance with this invention include, but are not limited to isothiazolones, glutaraldehydes, thiones, halogenated nitrilalkylamides, carbamates, halogenated alkyl nitrodioxanic, halogenated alkylhydantoins, halogenated nitroalkyldiols, thiocyanates, alkylphosphonium halides, guanidines, benzyl ammonium halides, alkylsulfonium methosulfates and the like. Table 1 provides a list of specific examples of these biocides and their typical active dosage amounts that have been found to be monitorable using the method of this invention.

**Table 1**

| Biocides Used as Cooling Water Treatments | |
|---|---|
| ppm Active | Active Ingredient |
| 1.5% | 5-chloro-2-methyl-4-isothiazoline-3-one 2-methyl-4-isothiazolin-3-one |
| 10% | methylene bis(thiocyanate) |
| 20% | tetrahydro-3,5-dimethyl-2H-1,3,5-thiadiazine-2-thione |
| 15% | sodium dimethyldithio carbamate |
| 15% | disodium ethylene-bis-dithiocarbamate |
| 12.5% | N-alkyl-dimethyl benzyl ammonium chloride |
| 2.14% | bis(tri-n-butyl) tinoxide |
| 20% | 2,2-dibromo-3-nitrilpropionamide |
| 45% | glutaraldehyde |
| 60% | 1-bromo-3-chloro-5,5-dimethylhydantoin |
| 27.4% | 1,3-dichloro-5,5-dimethylhydantoin |
| 10.6% | 1,3-dichloro-5-ethyl-5-methylhydantoin |

The individual concentrations of mixtures of two or more biocides may also be simultaneously monitored in accordance with the present invention.

A particularly preferred biocide combination comprises a mixture of glutaraldehyde and isothiazolones in a weight ratio of from 10:90 to 90:10 and is most preferably in a weight ratio of 1.5 to 10 of isothiazolones:glutaraldehyde, respectively. Suitable isothiazolones for use in this invention are commercially available from Rohm & Haas Company under the Kathon® trademark. In accordance with the present invention, the respective concentrations of both of these biocides can be directly and simultaneously monitored by determining the spectrum of the aqueous system containing the biocides, and then applying chemometrics algorithms to the spectrum. As is apparent, the application of chemometrics algorithms to a spectrum of an aqueous system is a powerful tool which provides the ability to simultaneously determine the concentrations of multiple components even in a complex matrix such as a cooling water system containing a plurality of biocides as well as other water treatment compositions or interferences.

Combinations of biocides with dispersants and/or biocide protectors may also be monitored in accordance with this invention. As used herein, biocide protector refers to a composition which inhibits the degradation of biocides in the presence of deleterious materials. For example, it is known that isothiazolone degrades under certain pH ranges or in the presence of iron metal. However, this degradation may be inhibited by the addition of one or more biocide protectors such as acetate, carbonates, chlorides, bromides, sulfates, phosphates, metal oxides, molybdates, chromates, zinc salts, copper salts, cadmium salts, dialkylthioureas, alkoxylated rosin amines, azoles, phosphonates, zinc dust, metal nitrates, or nitrites and the like, and mixtures thereof. This technology is more fully described in Canadian Patent Application 529,467, U.S. Patent 4,031,055 and U.S. Patent 3,820,795 which are incorporated herein in their entirety. In accordance with the present invention the concentration levels of biocides in combination with these biocide protectors and/or dispersants may also be directly and simultaneously monitored and quantified using the chemometrics algorithms hereinafter described.

Another embodiment of this invention is directed to a combination of monitoring methods to provide not only the concentration of one or more biocides in an aqueous system, but also the total biomass of living organisms in the system. The combined use of these technologies provides fast accurate tracking of biocide levels. This information is critical for determining system control parameters such as biocidal kill rates, effective biocide concentrations (and thereby avoid overdosing the biocides) as well as frequency of biocide addition. The method comprises directly determining the biocide concentration in accordance with the method of the present invention in combination with a bioassay technique such as an Adenosine triphosphate (ATP) test (as disclosed more fully in "Standard Methods for Examination of Water and Wastewater 17th Edition" (Washington, D.C.:American Public Health Association, 1989) pp 9-37 which is incorporated herein in its entirety), Deposit Accumulation Test System (DATS), a biofilm coupon as disclosed in U.S. Patent 5,051,359 (which is incorporated herein by reference in its entirety), infrared monitoring systems which monitor biofilm growth as a function of infrared absorbance and the like. These bioassays may be run continuously or intermittantly over a period of time. If the bioassays indicate an increase in microbial growth over time it is apparent that the current level of biocide is too low, and accordingly, the dosage amount should be increased.

Aqueous systems which are suitable for monitoring in accordance with the method of this invention generally include any aqueous systems where the system water is clear enough to obtain an absorbance or emission spectrum. These include, but are not limited to open or closed cooling water systems, process water systems such as e.g. pulp and paper making systems, air washers, metal working fluids, and the like. The system water is generally sampled in an area that is well mixed to assure that it is representative of this aqueous system. If the particular aqueous system is known to have relatively large amounts of particulate matter, it is advisable to filter the system water prior to obtaining its spectrum.

Generally, any commercial grade UV, visible and/or near infrared spectrometer may be used in accordance with this invention. For example, it is possible to use fixed wavelength detectors where discrete elements are placed at specific wavelengths which generally correspond to the absorbance or emission maxima for the particular water treatment composition. Charged coupled device (CCD) analyzers may also be used. It is preferred that the spectrometer have a resolution of at least 10 nm, preferably 2 nm and most preferably 1 nm.

A diode array spectrometer having a wavelength range of from 200 to 2500 nm is preferred for use in this invention and most preferably has a wavelength range of from 200 to 800 nm. Instrument stability is an important consideration when operating in areas with a high potential for electrical and mechanical noise. The spectrometer is preferably designed to operate at 40°C to eliminate any varying temperature effects.

The spectrometer may be used to monitor off-line samples, or in a preferred embodiment, is equipped with an on-line fiber optic probe. For on-line measurements a flow through optical chamber (optrode) is preferred. In these systems, light from a xenon flash lamp (or other suitable source) is transmitted to the optrode via a quartz fiber optic cable. The light is transmitted through the steam generator aqueous solution and collected in a second fiber optic cable which transmits the light to the diode array spectrometer. In the spectrometer the light is converted into an analog voltage for each pixel of the array. The array is then read by a computer and by subtracting a previously stored deionized water scan from the sample scan a true absorption spectrum is generated. The resultant spectrum is then processed by a chemometrics calibration algorithm to generate a quantitative multicomponent analysis for any and all of the water treatment compositions of interest.

Chemometrics is the application of statistical and pattern recognition techniques to chemical analysis. Quantitative estimates of chemical concentration in reagentless UV-vis-NIR spectroscopy are based on algorithms, the parameters of which are determined in calibration sequences called learning sets. Learning sets consist of a large number of known samples that are used to determine the parameters of the algorithms. The number of samples required depends on the complexity of the matrix, the number of spectroscopic interferences that are present, and the number of variables used in the algorithm. In general, the number of samples should be at least 10 times the number of independent variables employed. In the presence of known and unknown interferences, a multiple sample calibration averages out the effects of these interferences. The learning set solutions are prepared in a manner to typify the interferences and their variability that will be experienced in the steam generating system.

The invention preferably uses a multi-sample calibration based on either principle component regression analysis or rotated principle component analysis of absorbance or emissivity, and subsequent derivative data. The rotated principle component analysis method is most preferred and involves a rotation of the principle components which allows the concentration of all the relevant information for a particular analyte into a single rotated principle component. The use of rotated principle components enables one to detect weak UV-vis-NIR species that would not normally be quantifiable using more conventional chemometric techniques. Thus, the use of rotated principle components gives the invention the ability to detect weak UV-vis-NIR species that would normally not be quantifiable using more conventional chemometric techniques.

The most accurate calibration method for each analyte may be determined by selecting the particular method having the highest coefficient of determination (r²) value.

Without further elaboration, it is believed that one of ordinary skill in the art using the foregoing detailed description can use the present invention to its fullest extent. The following examples are provided to illustrate the present invention in accordance with the principles of this invention, but are not to be construed as limiting the invention in any way except as indicated in the appended claims. All parts and percentages are by weight unless otherwise indicated.

### Examples

In all of the examples given the following operating parameters, calibration methods and chemical techniques were employed.

### Operating parameters:

On-line analyzer.
Resolution 2 nm.
Solution path length 1.3 cm.
Operating temperature 40°C.
Static solution measurements.

### Chemometric Techniques

Learning set size (10-70) samples.
Wavelength range for calibration (30 wavelengths in the range 230-346 nm).
Calibration based on principle component regression of adsorbance, first derivative or second derivative.
Calibration based on rotated principle component on adsorbance spectrum, first derivative or second derivative.

### Chemical Referee Techniques

All analytical solutions were prepared to volumetric standards.
Referee techniques used were HPLC for 5-chloro-2-methyl-4-isothiazoline-3-one and a spectrophotoiodometric method for 2,2-dibromo-3-nitrilpropionamide.

### Example 1

This example demonstrates the ability to monitor biocides on-line. This experiment was run on a common cooling water microbiocide, 5-chloro-2-methyl-4-isothiazolin-3-one, in a simulated cooling tower (SCT) which comprised an automated 36 liter capacity pilot cooling tower equipped with an evaporative column, heat exchangers and controller. This controller monitors and controls various functions such as pH, conductivity, makeup and blowdown. The typical half-life of the system is 18 hours. The tower was treated with a standard corrosion/scale control product during the evaluations.

The analyzer was set-up so that water samples were removed from areas of good circulation and returned to the basin of the cooling tower. The analyzer's digital outputs were recorded by a commercial telecommunication program via a RS-232 connection.

A common cooling water biocide containing 5-chloro-2-methyl-3-isothiazolin-3-one and 2-methyl-4-isothiazolin-3-one was tested in the SCT unit with the analyzer monitoring the active constituents concentration. This biocide was chosen as a test biocide since it possesses absorbance characteristics suitable for the required chemometric algorithms and there is a High Pressure Liquid Chromatography (HPLC) reference method available to validate analyzer predictions. The HPLC method has an average relative error of 4.1% based on more than 20 control samples tested on various occasions.

The following SCT operating conditions were maintained during this trial:

| | |
|---|---|
| pH | 8.3 |
| Conductivity | 1.33 mmhos |
| Calcium Hardness | 500 ppm as CaCO₃ |
| Total Hardness | 830 ppm as CaCO₃ |
| M-Alkalinity | 60 ppm as CaCO₃ |
| Ortho-Phosphate | 0.6 ppm |
| Total Phosphate | 2.0 ppm |
| Temperature | 43°C (110°F) |

There are two types of learning set samples which can be used in calibration. Learning set samples can be taken directly from the cooling tower without further modification or background waters can be removed and spiked with the analyte of interest at various levels. The latter technique is often referred to as the standard addition method. The SCT Biocide #1 learning set included both types of calibration samples.

Background waters collected over a one week period were spiked with different levels of biocide. Some background waters were collected while being chlorinated and some with no chlorine present. Approximately 50 individual samples were made and scanned for calibration. Several different calibrations were made from the information obtained and evaluated using an off-line analysis program. The best calibrations were selected and uploaded to the analyzer computer to be used for on-line monitoring of the SCT run.

Four spikes of the isothiazolin-3-one mixture were made to the basin of the cooling tower to achieve 4, 12, 7 and 5 ppm as active biocide. After each spike of the isothiazolin-3-one, biocide levels were allowed to deplete to near 0 ppm before another spike was added. The analyzer was programmed to read every 20 minutes and the results were recorded by an external computer, while two to three grab samples were collected daily for HPLC analyses. These grab samples were stored in a cold room at 2-3°C prior to HPLC analysis to prevent any sample degradation.

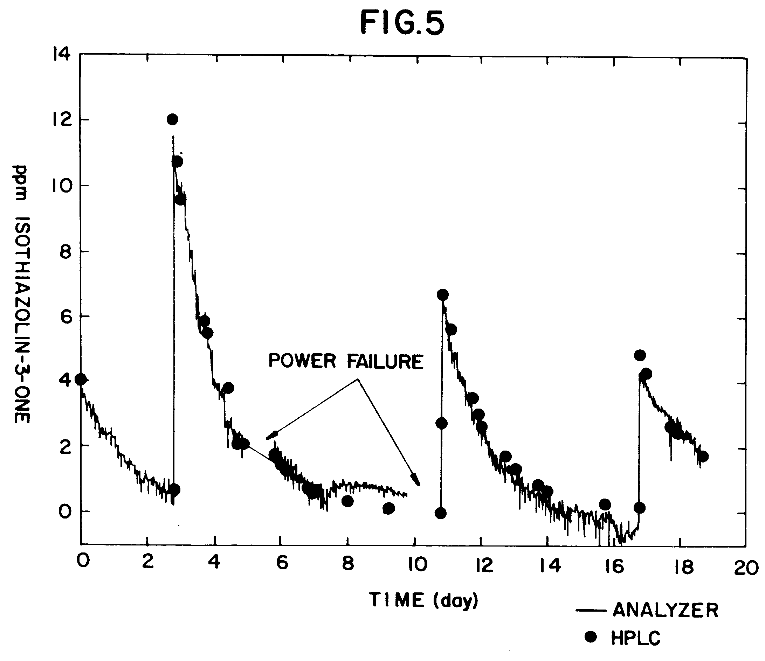

The analyzer readings (line plot) and HPLC results (filled circles) for total isothiazolin-3-one levels obtained over the nineteen day evaluation were in excellent agreement (see Figure 5). The analyzer output curve clearly indicates that each biocide spike was followed with the predicted exponential decay. No analyzer data is available for days 5 and 10 due to power failures caused by local storms.

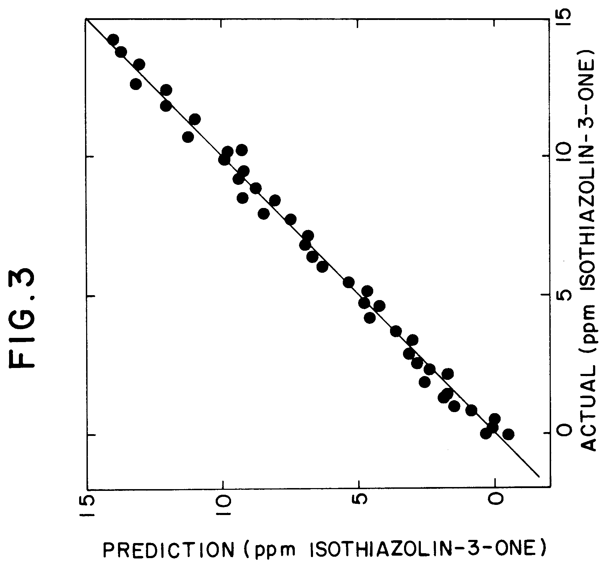

Figure 3 shows actual isothiazolin-3-one levels versus calibration predictions for the learning set used in the trial. The diagonal line in the figure represents the perfect match and the absolute error of a prediction is represented by the vertical distance between the line and the predicted point.

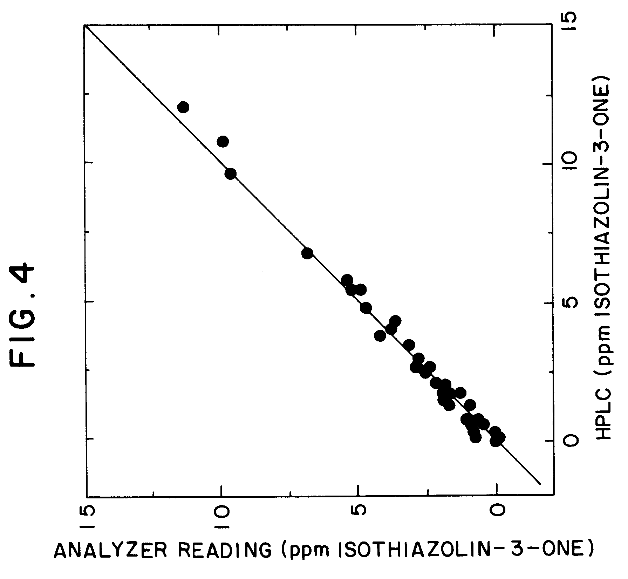

Figure 4 is a plot of HPLC results versus the corresponding analyzer readings for the trial grab samples. Although prediction error for Figure 4 is slightly larger than that obtained for the learning set, it is easily seen that the analyser can produce extremely reliable numbers in an on-line analysis situation. The calculated correlation coefficient (R²) for the data in Figure 4 is 0.99 where 1.0 represents the ideal.

### Example 2

This experiment demonstrated the ability to directly monitor another common cooling water microbiocide 2,2-dibromo-3-nitrilpropionamide (DBNPA) in the SCT unit with the analyzer monitoring active concentration. The absorbance pattern for DBNPA is quite different from that of the isothiazolin-3-one. An iodometric titration reference method for DBNPA was used to validate analyzer predictions.

The SCT rig operating conditions were modified slightly to extend the half-life of DBNPA in the cooling tower. The conditions were as follows:

| | |
|---|---|
| pH | 7.0 |
| Conductivity | 1.33 mmhos |
| Calcium Hardness | 570 ppm as CaCO₃ |
| Total Hardness | 950 ppm as CaCO₃ |
| M-Alkalinity | 10 ppm as CaCO₃ |
| Ortho-Phosphate | 0.4 ppm |
| Total Phosphate | 1.7 ppm |
| Temperature | 38°C (100°F) |

The DBNPA learning set consisted of 70 samples and was established in the same manner as described in the previous example.

Five DBNPA spikes were made to the basin of the cooling tower to achieve 15, 3, 20, 7 and 30 ppm as active DBNPA. After each spike, biocide levels were allowed to deplete to near 0 ppm before an additional spike was made. Again, the analyzer took a reading every 20 minutes and several grab samples were collected daily for immediate titration.

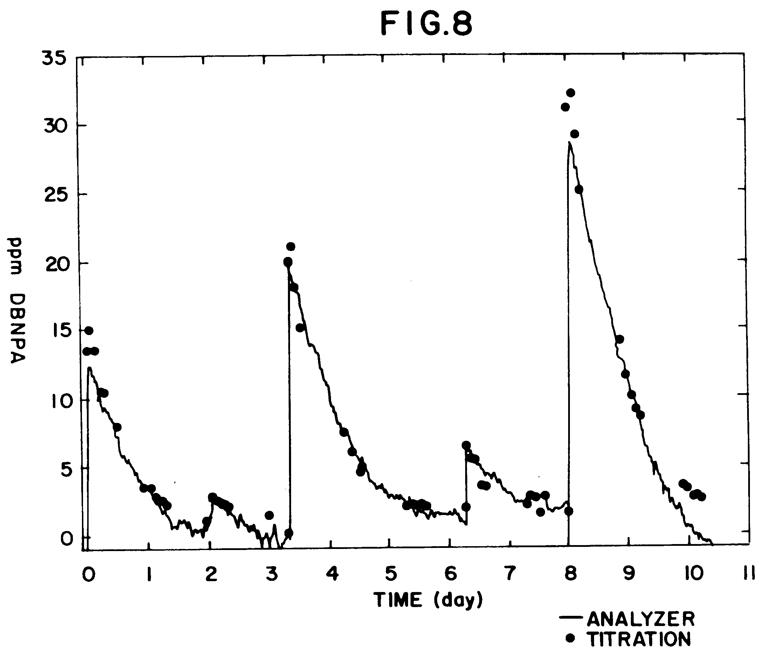

Figure 8 shows the corresponding analyser readings (line plot) and titration results (filled circles) over the ten day evaluation period. Shorter but consistent exponential decay patterns were observed following each spike of DBNPA when compared to those obtained in the isothiazolin-3-one trial. This difference may be related to the relative degradation rates of the two biocides. The analyzer showed very good predictions for the mid-concentration range and slightly lower predictions for the upper and lower concentration ranges.

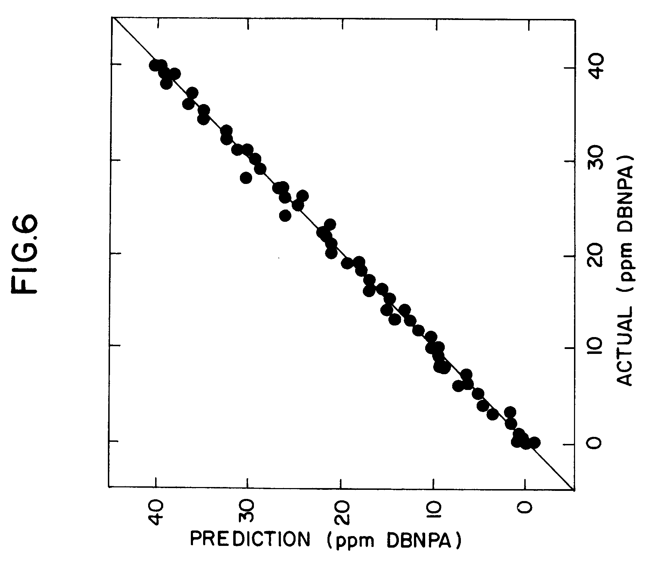

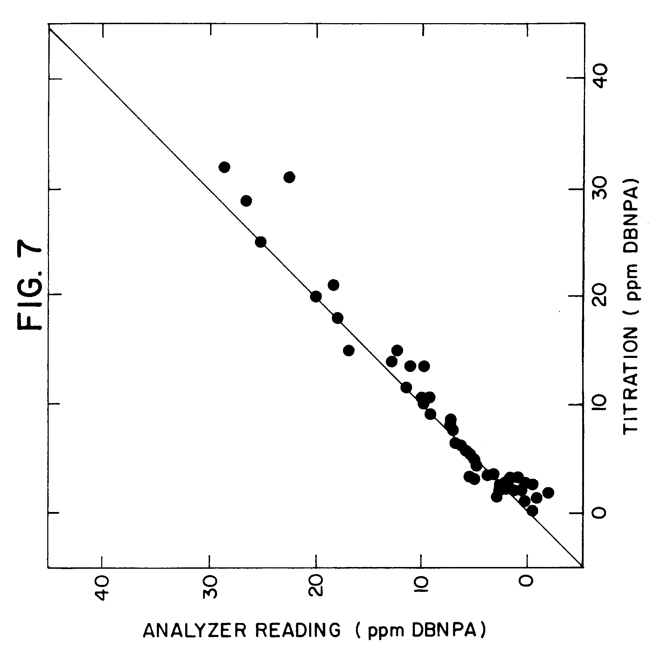

Learning set and SCT run plots are contained in Figures 6 and 7 and again it is evident that the analyzer can produce extremely reliable predictions of biocide concentrations. The R² value for the sensor readings and grab sample analyses is 0.94 (Figure 7).

## Claims

1. A method for directly measuring the concentrations of one or more biocides in aqueous systems comprising directly determining an absorbance or emission spectrum of the aqueous system containing the biocides in a wavelength range of from 200 to 2500 nm and applying chemometrics algorithms to the spectrum to determine the concentrations of the biocides.

2. A method according to Claim 1 wherein the aqueous system is selected from the group consisting of cooling water systems, metal working fluid systems, process water, pulp and papermaking water systems.

3. A method according to Claim 1 wherein the biocide is selected from the group consisting of glutaraldehyde, isothiazolones, nitrilpropionamides, thiocyanates, carbamates, quaternary ammonium chlorides, trialkyltinoxides, hydantoin, and mixtures thereof.

4. A method according to Claim 3 wherein the biocide comprises a mixture of glutaraldehyde and isothiazolone in a weight ratio of 5:95 to 95:5.

5. A method according to Claim 3 wherein the biocide comprises a mixture of glutaraldehyde and isothiazolone in a weight ratio of 10:90 to 90:10.

6. A method for determining effective biocide concentration in an aqueous system comprising a) determining the biocide concentration in accordance with Claim 1, b) determining total biomass levels with one or more bioassay and if the level of total biomass increases, increasing the biocide concentration in the system until the total biomass decreases or remains constant.
